# EUROPEAN PATENT APPLICATION

(11) **EP 3 486 317 A1**
(43) Date of publication of application: **22.05.2019**
(21) Application number: 17202022.4
(22) Date of filing: 16.11.2017
(51) Int. Cl.: C12N 9/02

(54) **ENANTIONSELECTIVE ENZYMATIC SULFOXIDATION OF CHIRAL ARYLSUFIDES**

(71) Applicant: Bayer Aktiengesellschaft, 51373 Leverkusen (DE)
(72) Inventor: Koch, Rainhard, 14532 Kleinmachnow (DE); Klaffl, Simon, 40597 Düsseldorf (DE); Spelberg, Markus, 40593 Düsseldorf (DE); Tischler, Dirk, 09599 Freiberg (DE); Schlömann, Michael, 09599 Freiberg (DE); Scholtissek, Anita, 09599 Freiberg (DE); Heine, Thomas, 09599 Freiberg (DE); Willrodt, Christian, 04105 Leipzig (DE); Bühler, Bruno, 04159 Leipzig (DE); Schmid, Andreas, 44229 Dortmund (DE)
(74) Representative: BIP Patents

(57) **Abstract**

What is described herein refers to isolated nucleic acid fragments encoding an oxygenase subunit (StyA) and a reductase subunit (StyB), wherein the polypeptide encoded for by the nucleotide sequence for the oxygenase subunit (StyA) and the nucleotide sequence for the reductase subunit (StyB) have activity towards chiral arylsulfides.

SEQUENCE LISTING

<110>  Bayer Aktiengesellschaft

<120>  Enantionselective enzymatic sulfoxidation of arylsufides

<130>  BTS173008

<160>  6

<170>  PatentIn version 3.5

<210>  1
<211>  1244
<212>  DNA
<213>  Acinetobacter baylyi

<400>  1
tccatatgcg tcgtatagca attgttggag cgggtcagtc tggattacag ctcggtttaa      60

gcctgttaga cacaggttat gatgtcacaa ttgtgaccaa ccgtaccgca gaccaaattc     120

gtcagggtaa ggtcatgtca agtcagtgta tgtttcatac cgctttgcaa actgaacgtg     180

atgttgggct caacttctgg gaagagcaat gtcccgctgt tgaaggaatt ggatttaccc     240

tggttagtcc agagacagga aaacctgcat tttcgtggag tgcacgtctt gagcgttatg     300

ctcaatcggt ggatcaacgc gtaaaaatgc cttactggat tgaagagttt gaacgtcgcg     360

gtggcaaact gattattcag gatgttggga ttgatgaact agaacaactg actaccgagt     420

atgaactggt gttgctggca gcaggtaagg gcgaagtggt gaaacagttt gtgcgtgatg     480

atgagcgcag cacattcgat aagccacagc gtgcgcttgc tttgacttat gtcacaggga     540

tgaaaccgat gtcgccgtat tcacgggtga cctttaatgt gattccgggc gttggcgagt     600

acttttgttt ccctgcactc accgtgacag gcccatgcga aattatggtg ttcgaaggga     660

ttccaggtgg gccaatggac tgctggcaag atgccaaaac gcctgagcaa catttgcaaa     720

tgagtaaaga cattctcaat acctatctgc cttgggaagc tgagcgttgt gaaaatattg     780

aaatcaccga tgcaggcggc tatttggctg gacgcttccc accgagcgtg cgtaaaccga     840

tactgacgct accatctggt cgtcaggtgt ttggtatggc agatgcgctg gtcgtgaatg     900

atccgattac gggtcaaggt tcaaacaacg ccgccaaatg cagcaagatt tattttgatg     960

ccattttagc gcatgacacg cagtctttta cgcctgaatg gatgcaacaa acctttgaac    1020

gttactggtc ttatgccgaa aaagtggtgg cttggaccaa cagcttactg gttccacctc    1080

agccacagat gattgatgta ttggccgcag caagccaaaa ccaagccatt gcctccacga    1140

ttgccaataa ctttgatgac cctcgtaatt tctctccgtg gtggtttgat gcagagcagg    1200

cacagcattt tatcgaatcg aaaagttgtc agaaagtggc ttaa                      1244

<210>  2
<211>  537

<212>  DNA
<213>  Acinetobacter baylyi

<400>  2
atgaatatta atacatcaca tgagctcggt ttaaagccga ttgatacaga aaatccgaga      60

gaaatccgaa atttacttgg acagtttgca actggcgtaa ccgtgattac cacgcgtggt     120

cgtgatggac gaaaaatcgg aatgaccgcc aattcatttt catcattatc acttgatcca     180

cccttaattt tgtggagttt gtcaaaaact gcaccgagtc tgccagactt tactgaggcg     240

gaatatttcg cgattcacat gctggctcaa gagcatcatt cactttctgg acattttgca     300

cggggttcag aagacaaatt cgccagtatt gcacatcgtg aatgtgaacg tggcctacct     360

ttgcttgaag atgtacttgc gacattggtg tgtaaaaaca ttaaccaata tgaaggcggt     420

gatcacctga tttttatcgg tcagattgag cattatcaac aacgcatcgg tgagccattg     480

gtttttcatg cgggtaaata tcgtattgca gcagagcatc cagagctcag tgcataa        537


<210>  3
<211>  1083
<212>  DNA
<213>  Artificial Sequence

<220>
<223>  lacI gene coding for lac repressor

<400>  3
tcactgcccg ctttccagtc gggaaacctg tcgtgccagc tgcattaatg aatcggccaa      60

cgcgcgggga gaggcggttt gcgtattggg cgccagggtg gtttttcttt tcaccagtga     120

gacgggcaac agctgattgc ccttcaccgc ctggccctga gagagttgca gcaagcggtc     180

cacgctggtt tgccccagca ggcgaaaatc ctgtttgatg gtggttaacg gcgggatata     240

acatgagctg tcttcggtat cgtcgtatcc cactaccgag atgtccgcac caacgcgcag     300

cccggactcg gtaatggcgc gcattgcgcc cagcgccatc tgatcgttgg caaccagcat     360

cgcagtggga acgatgccct cattcagcat ttgcatggtt tgttgaaaac cggacatggc     420

actccagtcg ccttcccgtt ccgctatcgg ctgaattgat ttgcgagtga gatatttatg      480

ccagccagcc agacgcagac gcgccgagac agaacttaat gggcccgcta acagcgcgat     540

ttgctggtga cccaatgcga ccagatgctc cacgcccagt cgcgtaccgt cttcatggga     600

gaaaataata ctgttgatgg gtgtctggtc agagacatca agaaataacg ccggaacatt     660

agtgcaggca gcttccacag caatggcatc ctggtcatcc agcggatagt taatgatcag     720

cccactgacg cgttgcgcga gaagattgtg caccgccgct ttacaggctt cgacgccgct     780

tcgttctacc atcgacacca ccacgctggc acccagttga tcggcgcgag atttaatcgc     840

cgcgacaatt tgcgacggcg cgtgcagggc cagactggag gtggcaacgc caatcagcaa     900

cgactgtttg cccgccagtt gttgtgccac gcggttggga atgtaattca gctccgccat     960

cgccgcttcc actttttccc gcgttttcgc agaaacgtgg ctggcctggt tcaccacgcg 1020

ggaaacggtc tgataagaga caccggcata ctctgcgaca tcgtataacg ttactggttt 1080

cac 1083

<210> 4
<211> 8067
<212> DNA
<213> Artificial Sequence

<220>
<223> DNA sequence of the plasmid pCW077_pStyAB_ADP1_lac

<400> 4
cgacctgcag ccaagcttct gttttggcgg atgagagaag attttcagcc tgatacagat 60

taaatcagaa cgcagaagcg gtctgataaa acagaatttg cctggcggca gtagcgcggt 120

ggtcccacct gaccccatgc cgaactcaga agtgaaacgc cgtagcgccg atggtagtgt 180

ggggtctccc catgcgagag tagggaactg ccaggcatca aataaaacga aaggctcagt 240

cgaaagactg ggcctttcgt tttatctgtt gtttgtcggt gaacgctctc ctgagtagga 300

caaatccgcc gggagcggat ttgaacgttg cgaagcaacg gcccggaggg tggcgggcag 360

gacgcccgcc ataaactgcc aggcatcaaa ttaagcagaa ggccatcctg acggatggcc 420

tttttgcgtt tctacaaact cttttgttta tttttctaaa tacattcaaa tatgtatccg 480

ctcatgagac aataaccctg ataaatgctt caataatgca gcctgaaagg caggccgggc 540

cgtggtggcc acggcctcta ggccagatcc agcggcatct gggttagtcg agcgcgggcc 600

gcttcccatg tctcaccagg gcgagcctgt ttcgcgatct cagcatctga aatcttcccg 660

gccttgcgct tcgctggggc cttacccacc gccttggcgg gcttcttcgg tccaaaactg 720

aacaacagat gtgtgacctt gcgcccggtc tttcgctgcg cccactccac ctgtagcggg 780

ctgtgctcgt tgatctgcgt cacggctgga tcaagcactc gcaacttgaa gtccttgatc 840

gagggatacc ggccttccag ttgaaaccac tttcgcagct ggtcaatttc tatttcgcgc 900

tggccgatgc tgtcccattg catgagcagc tcgtaaagcc tgatcgcgtg ggtgctgtcc 960

atcttggcca cgtcagccaa ggcgtatttg gtgaactgtt tggtgagttc cgtcaggtac 1020

ggcagcatgt ctttggtgaa cctgagttct acacggccct caccctcccg gtagatgatt 1080

gtttgcaccc agccggtaat catcacactc ggtcttttcc ccttgccatt gggctcttgg 1140

gttaaccgga cttcccgccg tttcaggcgc agggccgctt ctttgagctg gttgtaggaa 1200

gattcgatag ggacacccgc catcgtcgct atgtcctccg ccgtcactga atacatcact 1260

tcatcggtga caggctcgct cctcttcacc tggctaatac aggccagaac gatccgctgt 1320

tcctgaacac tgaggcgata cgcggcctcg accagggcat tgcttttgta aaccattggg 1380

ggtgaggcca cgttcgacat tccttgtgta taaggggaca ctgtatctgc gtcccacaat 1440

acaacaaatc cgtcccttta caacaacaaa tccgtccctt cttaacaaca aatccgtccc 1500

ttaatggcaa caaatccgtc cctttttaaa ctctacaggc cacggattac gtggcctgta 1560

gacgtcctaa aaggtttaaa agggaaaagg aagaaaaggg tggaaacgca aaaaacgcac 1620

cactacgtgg ccccgttggg gccgcatttg tgcccctgaa ggggcggggg aggcgtctgg 1680

gcaatccccg ttttaccagt cccctatcgc cgcctgagag ggcgcaggaa gcgagtaatc 1740

agggtatcga ggcggattca cccttggcgt ccaaccagcg gcaccagcgg cgcctgagag 1800

gcgaattgac ataagcctgt tcggttcgta aactgtaatg caagtagcgt atgcgctcac 1860

gcaactggtc cagaaccttg accgaacgca gcggtggtaa cggcgcagtg gcggttttca 1920

tggcttgtta tgactgtttt tttgtacagt ctatgcctcg ggcatccaat cgatgggaag 1980

ccctgcaaag taaactggat ggctttcttg ccgccaagga tctgatggcg caggggatca 2040

agatctgatc aagagacagg atgaggatcg tttcgcatga ttgaacaaga tggattgcac 2100

gcaggttctc cggccgcttg ggtggagagg ctattcggct atgactgggc acaacagaca 2160

atcggctgct ctgatgccgc cgtgttccgg ctgtcagcgc aggggcgccc ggttcttttt 2220

gtcaagaccg acctgtccgg tgccctgaat gaactgcagg acgaggcagc gcggctatcg 2280

tggctggcca cgacgggcgt tccttgcgca gctgtgctcg acgttgtcac tgaagcggga 2340

agggactggc tgctattggg cgaagtgccg gggcaggatc tcctgtcatc tcaccttgct 2400

cctgccgaga aagtatccat catggctgat gcaatgcggc ggctgcatac gcttgatccg 2460

gctacctgcc cattcgacca ccaagcgaaa catcgcatcg agcgagcacg tactcggatg 2520

gaagccggtc ttgtcgatca ggatgatctg gacgaagagc atcaggggct cgcgccagcc 2580

gaactgttcg ccaggctcaa ggcgcgcatg cccgacggcg aggatctcgt cgtgacccat 2640

ggcgatgcct gcttgccgaa tatcatggtg gaaaatggcc gcttttctgg attcatcgac 2700

tgtggccggc tgggtgtggc ggaccgctat caggacatag cgttggctac ccgtgatatt 2760

gctgaagagc ttggcggcga atgggctgac cgcttcctcg tgctttacgg tatcgccgct 2820

cccgattcgc agcgcatcgc cttctatcgc cttcttgacg agttcttctg agcgggactc 2880

tggggttcga aatgaccgac caatcgattg gtaactgtca gaccaagttt actcatatat 2940

actttagatt gatttaaaac ttcattttta atttaaaagg atctaggtga agatcctttt 3000

tgataatctc atgaccaaaa tcccttaacg tgagttttcg ttccactgag cgtcagaccc 3060

cgtagaaaag atcaaaggat cttcttgaga tccttttttt ctgcgcgtaa tctgctgctt 3120

gcaaacaaaa aaaccaccgc taccagcggt ggtttgtttg ccggatcaag agctaccaac 3180

tctttttccg aaggtaactg gcttcagcag agcgcagata ccaaatactg tccttctagt 3240

gtagccgtag ttaggccacc acttcaagaa ctctgtagca ccgcctacat acctcgctct 3300

gctaatcctg ttaccagtgg ctgctgccag tggcgataag tcgtgtctta ccgggttgga 3360

ctcaagacga tagttaccgg ataaggcgca gcggtcgggc tgaacggggg gttcgtgcac 3420

acagcccagc ttggagcgaa cgacctacac cgaactgaga tacctacagc gtgagctatg 3480

agaaagcgcc acgcttcccg aagggagaaa ggcggacagg tatccggtaa gcggcagggt 3540

cggaacagga gagcgcacga gggagcttcc agggggaaac gcctggtatc tttatagtcc 3600

tgtcgggttt cgccacctct gacttgagcg tcgatttttg tgatgctcgt caggggggcg 3660

gagcctatgg aaaaacgcca gcaacgcggc ctttttacgg ttcctggcct tttgctggcc 3720

ttttgctcac atgttctttc ctgcgttatc ccctgattct gtggataacc gtattaccgc 3780

ctttgagtga gctgataccg ctcgccgcag ccgaacgacc gagcgcagcg agtcagtgag 3840

cgaggaagcg gaagagcgcc tgatgcggta ttttctcctt acgcatctgt gcggtatttc 3900

acaccgcata ggggatctcc aatcgtgcct tggcgcagcg acagccctcg gtcccccaga 3960

tagccattga tcctctctcg cctgtcccct cagttcagta atttcctgca tttgcctgtt 4020

tccagtcggt agatattcca caaaacagca gggaagcagc gcttttccgc tgcataaccc 4080

tgcttcgggg tcattatagc gattttttcg gtatatccat cctttttcgc acgatataca 4140

ggattttgcc aaagggttcg tgtagacttt ccttggtgta tccaacggcg tcagccgggc 4200

aggataggtg aagtaggccc acccgcgagc gggtgttcct tcttcactgt cccttattcg 4260

cacctggcgg tgctcaacgg gaatcctgct ctgcgaggct ggccggctac cgccggcgta 4320

acagatgagg gcaagcggat ggctgatgaa accaagccaa ccaggaaggg cagcccacct 4380

atcaaggtgt actgccttcc agacgaacga agagcgattg aggaaaaggc ggcggcggcc 4440

ggcatgagcc tgtcggccta cctgctggcc gtcggccagg gctacaaaat cacgggcgtc 4500

gtggactatg agctcgagta tacttttcac tatatcactt aatgccgatt attttctaga 4560

aattctcatg ttagtcatgc ccgcgcccca ccggaaggag ctgactgggt tgaaggctct 4620

caagggcatc ggtcgagatc ccggtgccta atgagtgagc taacttacat taattgcgtt 4680

gcgctcactg cccgctttcc agtcgggaaa cctgtcgtgc cagctgcatt aatgaatcgg 4740

ccaacgcgcg gggagaggcg gtttgcgtat tgggcgccag ggtggttttt cttttcacca 4800

gtgagacggg caacagctga ttgcccttca ccgcctggcc ctgagagagt tgcagcaagc 4860

ggtccacgct ggtttgcccc agcaggcgaa aatcctgttt gatggtggtt aacggcggga 4920

tataacatga gctgtcttcg gtatcgtcgt atcccactac cgagatgtcc gcaccaacgc 4980

gcagcccgga ctcggtaatg gcgcgcattg cgcccagcgc catctgatcg ttggcaacca 5040

gcatcgcagt gggaacgatg ccctcattca gcatttgcat ggtttgttga aaaccggaca 5100

tggcactcca gtcgccttcc cgttccgcta tcggctgaat ttgattgcga gtgagatatt 5160

tatgccagcc agccagacgc agacgcgccg agacagaact taatgggccc gctaacagcg 5220

cgatttgctg gtgacccaat gcgaccagat gctccacgcc cagtcgcgta ccgtcttcat 5280

gggagaaaat aatactgttg atgggtgtct ggtcagagac atcaagaaat aacgccggaa 5340

cattagtgca ggcagcttcc acagcaatgg catcctggtc atccagcgga tagttaatga 5400

tcagcccact gacgcgttgc gcgagaagat tgtgcaccgc cgctttacag gcttcgacgc 5460

cgcttcgttc taccatcgac accaccacgc tggcacccag ttgatcggcg cgagatttaa 5520

tcgccgcgac aatttgcgac ggcgcgtgca gggccagact ggaggtggca acgccaatca 5580

gcaacgactg tttgcccgcc agttgttgtg ccacgcggtt gggaatgtaa ttcagctccg 5640

ccatcgccgc ttccactttt tcccgcgttt tcgcagaaac gtggctggcc tggttcacca 5700

cgcgggaaac ggtctgataa gagacaccgg catactctgc gacatcgtat aacgttactg 5760

gtttcacatt caccaccctg aattgactct cttccgggcg ctatcatgcc ataccgcgaa 5820

aggttttgcg ccattcgatg gtgtccggga tctcgacgct ctcccttatg cgactcctgc 5880

attaggaagc agcccagtag taggttgagg ccgttgagca ccgccgccgc aaggaatggt 5940

gtcgtcgccg cacttatgac tgtcttcttt atcatgcaac tcgtaggaca ggtgccggca 6000

gcgcccaaca gtcccccggc cacggggcct gtctcggtcg atcattcagc ccggctcata 6060

gatatgcggg cagtgagcgc aacgcaatta atgtaagtta gctcactcat taggcacccc 6120

aggcttgaca ctttatgctt ccggctcgta taatgtgtgg aattgtgagc ggataacaat 6180

aacaatttca cacaggatct aggaaccagt actggagaat tccatatgcg tcgtatagca 6240

attgttggag cgggtcagtc tggattacag ctcggtttaa gcctgttaga cacaggttat 6300

gatgtcacaa ttgtgaccaa ccgtaccgca gaccaaattc gtcagggtaa ggtcatgtca 6360

agtcagtgta tgtttcatac cgctttgcaa actgaacgtg atgttgggct caacttctgg 6420

gaagagcaat gtcccgctgt tgaaggaatt ggatttaccc tggttagtcc agagacagga 6480

aaacctgcat tttcgtggag tgcacgtctt gagcgttatg ctcaatcggt ggatcaacgc 6540

gtaaaaatgc cttactggat tgaagagttt gaacgtcgcg gtggcaaact gattattcag 6600

gatgttggga ttgatgaact agaacaactg actaccgagt atgaactggt gttgctggca 6660

gcaggtaagg gcgaagtggt gaaacagttt gtgcgtgatg atgagcgcag cacattcgat 6720

aagccacagc gtgcgcttgc tttgacttat gtcacaggga tgaaaccgat gtcgccgtat 6780

tcacgggtga cctttaatgt gattccgggc gttggcgagt acttttgttt ccctgcactc 6840

accgtgacag gcccatgcga aattatggtg ttcgaaggga ttccaggtgg gccaatggac 6900

tgctggcaag atgccaaaac gcctgagcaa catttgcaaa tgagtaaaga cattctcaat 6960

acctatctgc cttgggaagc tgagcgttgt gaaaatattg aaatcaccga tgcaggcggc 7020

tatttggctg gacgcttccc accgagcgtg cgtaaaccga tactgacgct accatctggt 7080

cgtcaggtgt ttggtatggc agatgcgctg gtcgtgaatg atccgattac gggtcaaggt 7140

tcaaacaacg ccgccaaatg cagcaagatt tattttgatg ccattttagc gcatgacacg 7200

cagtctttta cgcctgaatg gatgcaacaa acctttgaac gttactggtc ttatgccgaa 7260

aaagtggtgg cttggaccaa cagcttactg gttccacctc agccacagat gattgatgta 7320

ttggccgcag caagccaaaa ccaagccatt gcctccacga ttgccaataa ctttgatgac 7380

cctcgtaatt tctctccgtg gtggtttgat gcagagcagg cacagcattt tatcgaatcg 7440

aaaagttgtc agaaagtggc ttaagcggcc gcacttaagt tacgcgtgga taggagatat 7500

catatgaata ttaatacatc acatgagctc ggtttaaagc cgattgatac agaaaatccg 7560

agagaaatcc gaaatttact tggacagttt gcaactggcg taaccgtgat taccacgcgt 7620

ggtcgtgatg gacgaaaaat cggaatgacc gccaattcat tttcatcatt atcacttgat 7680

ccacccttaa ttttgtggag tttgtcaaaa actgcaccga gtctgccaga ctttactgag 7740

gcggaatatt tcgcgattca catgctggct caagagcatc attcactttc tggacatttt 7800

gcacggggtt cagaagacaa attcgccagt attgcacatc gtgaatgtga acgtggccta 7860

cctttgcttg aagatgtact tgcgacattg gtgtgtaaaa acattaacca atatgaaggc 7920

ggtgatcacc tgatttttat cggtcagatt gagcattatc aacaacgcat cggtgagcca 7980

ttggtttttc atgcgggtaa atatcgtatt gcagcagagc atccagagct cagtgcataa 8040

catatgcttg gcgcgcccgg gatccgt 8067

<210> 5
<211> 412
<212> PRT
<213> Acinetobacter baylyi

<400> 5

Met Arg Arg Ile Ala Ile Val Gly Ala Gly Gln Ser Gly Leu Gln Leu
1               5                   10                  15

Gly Leu Ser Leu Leu Asp Thr Gly Tyr Asp Val Thr Ile Val Thr Asn
            20                  25                  30

Arg Thr Ala Asp Gln Ile Arg Gln Gly Lys Val Met Ser Ser Gln Cys
        35                  40                  45

Met Phe His Thr Ala Leu Gln Thr Glu Arg Asp Val Gly Leu Asn Phe
    50                  55                  60

Trp Glu Glu Gln Cys Pro Ala Val Glu Gly Ile Gly Phe Thr Leu Val
65                  70                  75                  80

Ser Pro Glu Thr Gly Lys Pro Ala Phe Ser Trp Ser Ala Arg Leu Glu
                85                  90                  95

Arg Tyr Ala Gln Ser Val Asp Gln Arg Val Lys Met Pro Tyr Trp Ile
            100                 105                 110

Glu Glu Phe Glu Arg Arg Gly Gly Lys Leu Ile Ile Gln Asp Val Gly
        115                 120                 125

Ile Asp Glu Leu Glu Gln Leu Thr Thr Glu Tyr Glu Leu Val Leu Leu
    130                 135                 140

Ala Ala Gly Lys Gly Glu Val Val Lys Gln Phe Val Arg Asp Asp Glu
145                 150                 155                 160

Arg Ser Thr Phe Asp Lys Pro Gln Arg Ala Leu Ala Leu Thr Tyr Val
                165                 170                 175

Thr Gly Met Lys Pro Met Ser Pro Tyr Ser Arg Val Thr Phe Asn Val
            180                 185                 190

Ile Pro Gly Val Gly Glu Tyr Phe Cys Phe Pro Ala Leu Thr Val Thr
        195                 200                 205

Gly Pro Cys Glu Ile Met Val Phe Glu Gly Ile Pro Gly Gly Pro Met
    210                 215                 220

Asp Cys Trp Gln Asp Ala Lys Thr Pro Glu Gln His Leu Gln Met Ser
225                 230                 235                 240

Lys Asp Ile Leu Asn Thr Tyr Leu Pro Trp Glu Ala Glu Arg Cys Glu
                245                 250                 255

Asn Ile Glu Ile Thr Asp Ala Gly Gly Tyr Leu Ala Gly Arg Phe Pro
            260                 265                 270

Pro Ser Val Arg Lys Pro Ile Leu Thr Leu Pro Ser Gly Arg Gln Val
        275                 280                 285

Phe Gly Met Ala Asp Ala Leu Val Val Asn Asp Pro Ile Thr Gly Gln
    290                 295                 300

Gly Ser Asn Asn Ala Ala Lys Cys Ser Lys Ile Tyr Phe Asp Ala Ile
305                 310                 315                 320

Leu Ala His Asp Thr Gln Ser Phe Thr Pro Glu Trp Met Gln Gln Thr
                325                 330                 335

Phe Glu Arg Tyr Trp Ser Tyr Ala Glu Lys Val Val Ala Trp Thr Asn
            340                 345                 350

Ser Leu Leu Val Pro Pro Gln Pro Gln Met Ile Asp Val Leu Ala Ala
        355                 360                 365

Ala Ser Gln Asn Gln Ala Ile Ala Ser Thr Ile Ala Asn Asn Phe Asp
    370                 375                 380

Asp Pro Arg Asn Phe Ser Pro Trp Trp Phe Asp Ala Glu Gln Ala Gln
385                 390                 395                 400

His Phe Ile Glu Ser Lys Ser Cys Gln Lys Val Ala
                405                 410

<210> 6
<211> 178
<212> PRT
<213> Acinetobacter baylyi

<400> 6

Met Asn Ile Asn Thr Ser His Glu Leu Gly Leu Lys Pro Ile Asp Thr
1               5                   10                  15

Glu Asn Pro Arg Glu Ile Arg Asn Leu Leu Gly Gln Phe Ala Thr Gly
            20                  25                  30

Val Thr Val Ile Thr Thr Arg Gly Arg Asp Gly Arg Lys Ile Gly Met
        35                  40                  45

Thr Ala Asn Ser Phe Ser Ser Leu Ser Leu Asp Pro Pro Leu Ile Leu
    50                  55                  60

Trp Ser Leu Ser Lys Thr Ala Pro Ser Leu Pro Asp Phe Thr Glu Ala
65                  70                  75                  80

Glu Tyr Phe Ala Ile His Met Leu Ala Gln Glu His His Ser Leu Ser
                85                  90                  95

Gly His Phe Ala Arg Gly Ser Glu Asp Lys Phe Ala Ser Ile Ala His
            100                 105                 110

Arg Glu Cys Glu Arg Gly Leu Pro Leu Leu Glu Asp Val Leu Ala Thr
        115                 120                 125

Leu Val Cys Lys Asn Ile Asn Gln Tyr Glu Gly Gly Asp His Leu Ile
    130                 135                 140

Phe Ile Gly Gln Ile Glu His Tyr Gln Gln Arg Ile Gly Glu Pro Leu
145                 150                 155                 160

Val Phe His Ala Gly Lys Tyr Arg Ile Ala Ala Glu His Pro Glu Leu
                165                 170                 175

Ser Ala

## Description

Chiral sulfoxides can be potentially used as buildings blocks, chiral auxiliaries or active pharmaceutical ingredients. Due to their chiral nature and the fact, that the potency of some enantiomers is higher than of their antipode (e.g. esomeprazole), asymmetric synthesis is required. This can for example be achieved via the preparative separation of the enantiomers. However, this synthesis route is time consuming and higher yields would be desirable.

Thus, it was the object of the current invention to devise an alternative more efficient and more cost effective way of providing pure enantiomers of sulfoxides.

The invention achieves this object by provision of an isolated nucleic acid fragment encoding an oxygenase subunit (StyA) and an isolated nucleic acid fragment encoding a reductase subunit (StyB), wherein the polypeptides encoded for by the nucleotide sequences for the oxygenase subunit (StyA) and the nucleotide sequence for the reductase subunit (StyB) together have activity towards chiral arylsulfides and wherein said nucleic acid fragments are selected from the group
a) nucleic acid fragment comprising a nucleotide sequence of at least 80% sequence identity to SEQ ID NO 1 and a nucleotide sequence of at least 80% sequence identity to SEQ ID NO 2
b) nucleic acid fragment comprising a sequence complementary to SEQ ID NO 1 and a nucleic acid fragment comprising a sequence complementary to SEQ ID NO 2
c) nucleic acid fragment comprising a sequence which specifically hybridizes to said nucleic acid fragment of a) or said complementary of b).

Surprisingly, it was found that the oxygenase subunit (StyA) and the reductase subunit (StyB) encoded for by the isolated nucleic acid fragments described herein together catalyze the sulfoxide formation - i.e. the oxidation of a sulfide - in chiral aryl sulfides including heteroaromatic sulfides with high activity and high enantiomeric excess.

Without wishing to be bound by theory it is assumed that the oxygenase subunit (StyA) and the reductase subunit (StyB) encoded for by the isolated nucleic acid fragments described herein represent a styrene monooxygenase.

As used herein the term "styrene monooxygenase" (used synonymously with "SMO") refers to an enzyme that belongs to the group of E1-Typ Monooxygenase and is dependent on FAD as cofactor. Styrene monooxygenases (SMOs) are thought to be a class of enzymes performing a selective oxygenation of the vinyl side chain of styrene to styrene oxide or to oxidize indol (Sadauskas et al. 2017). Like most SMOs described so far also the polypetides described herein form a two-component system. The two components, a single oxidase and a single FAD reductase, are encoded by two separate genes, StyA and StyB, respectively. The reductase (StyB) solely uses NADH to reduce the FAD cofactor which is then utilized by the oxidase (StyA) to activate molecular oxygen and to catalyze the oxidation.

The inventors of the current invention have shown for the first time that chiral arylsulfoxides - i.e. in this case only the S-enantiomer of the oxidized compound according to formula I - can be generated efficiently enough, enantiomerically pure enough and in suitable quantities for synthesis on a production scale using a two component SMO i.e. the polypeptides described herein.

This is the case, even though styrene monooxygenase of other organism have been described previously (Tischler et al. 2009, Tischler et al. 2012).

The term "comprising" is to be interpreted as specifying the presence of the stated parts, steps or components, but does not exclude the presence of one or more additional parts, steps or components.

It is understood that when referring to a word in the singular, the plural is also included herein. Thus, reference to an element by the indefinite article "a" or "an" does not exclude the possibility that more than one of the element is present, unless the context clearly requires that there be one and only one of the elements. The indefinite article "a" or "an" thus usually means "at least one".

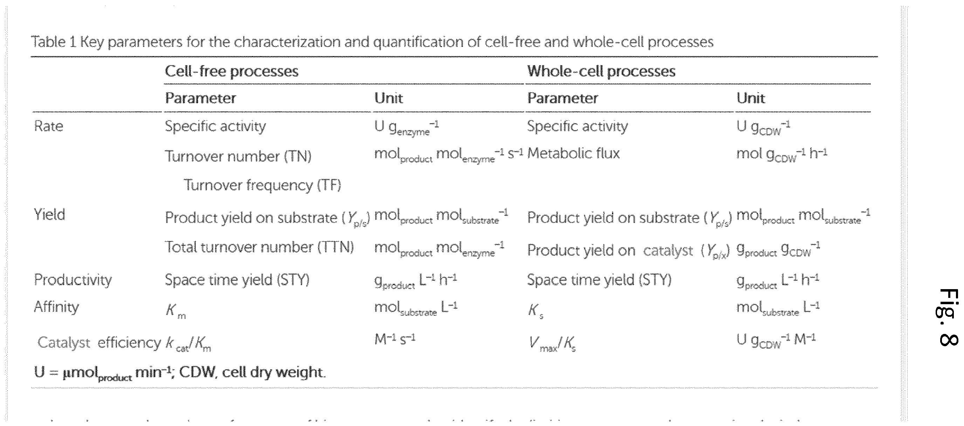

In general parameters determining catalyst and process efficiency are depicted in FIG 8.

A common unit to measure enzyme activity of whole cells is U / gcdw where U stands for unit i.e. for µmol/min. In other words if a biocatalyst has an activity of 1 U it generates 1 µmol of product per minute. As used herein the unit of enzyme activity refers to the employed biomass i.e. gramm cell dry weight (gcdw). Thus, a value of 450 U/gcdw means that 1 gramm dried cells (when the cells were still alive) catalyzed a reaction with 450 µmol/min and hence generated 450 µmol product per minute.

A reliably assertion of the suitability of a given enzyme can be made using the parameters: substrate conversion, product formation, enantiomeric excess (ee) and space time yield (STY). As used herein the term "space time yield" refers to the yield generated by a given amount of cells e.g. cultured in a culture vessel in a defined time. For example the space time yield seen in the experiments performed below was around 70g/l in 2,3 hours.

The term "oxygenase" as used herein refers to an enzyme that oxidizes a substrate by transferring an oxygen molecule to it.

The term "reductase" as used herein refers to an enzyme which transfers electrons to an oxygenase.

The term "nucleic acid molecule" is intended to indicate any single- or double stranded nucleic acid molecule comprising DNA (cDNA and/or genomic DNA), RNA (preferably mRNA), PNA, LNA and/or Morpholino.

As used herein the term "nucleic acid fragment" refers to an isolated nucleic acid molecule.

As used herein the term "gene" means a DNA sequence made up of nucleotides comprising a region (transcribed region), which is transcribed into an RNA molecule (e.g. directly into a mRNA without intron sequences) in a cell, operably linked to regulatory regions capable of regulating the expression of the polypeptide. A gene may thus comprise several operably linked sequences, such as untranslated regulatory regions (e.g. a promoter, enhancer, repressor), a 5' leader sequence comprising e.g. sequences involved in translation initiation, a (protein) coding region (cDNA or genomic DNA) and a 3' non-translated sequence comprising e.g. transcription termination sites.

As used herein the term "expression of a gene" or "gene expression" refers to the process wherein a DNA region (the coding region), which is operably linked to appropriate regulatory regions, particularly a promoter, is transcribed into an mRNA molecule. The mRNA molecule is then processed further (by post-transcriptional processes) within the cell, e.g. by translation initiation and translation into an amino acid chain (polypeptide), and translation termination by translation stop codons.

As used herein the term "Wild type" (also written "wildtype" or "wild-type"), refers to a typical form of a gene as it most commonly occurs in nature.

As used herein the term "polypeptide" refers to any peptide, polypeptide, oligopeptide or protein. A polypeptide consists of consecutive amino acids, which are linked by peptide bonds. The polymer may be linear or branched, it may comprise modified amino acids, and it may be interrupted by non-amino acids. The polypeptide may be human, non- human, and an artificial or chemical mimetic of a corresponding naturally occurring amino acid, as well as naturally occurring amino acid polymers and non- naturally occurring amino acid polymers. The term also encompasses an amino acid polymer that has been modified by either natural processes or by chemical modifications; for example, by disulfide bond formation, glycosylation, lipidation, acetylation, acylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

As used herein the term "sequence identity" of two related nucleotide or amino acid sequences, expressed as a percentage, refers to the number of positions in the two optimally aligned sequences which have identical residues (x 100) divided by the number of positions compared. A gap, i.e., a position in an alignment where a residue is present in one sequence but not in the other, is regarded as a position with non-identical residues. The "optimal alignment" of two sequences is found by aligning the two sequences over the entire length. In other words if two identical sequences are aligned the sequence identity value would be 100%.

Aligned sequences of nucleotide or amino acid residues are typically represented as rows within a matrix. Gaps are inserted between the residues so that identical or similar characters are aligned in successive columns.

In order to determine the sequence identity the Needleman and Wunsch global alignment algorithm (Needleman and Wunsch, 1970, J Mol Biol 48(3):443-53) of The European Molecular Biology Open Software Suite (EMBOSS, Rice et al., 2000, Trends in Genetics 16(6): 276- 277; see e.g. http://www.ebi.ac.uk/emboss/align/index.html) using default settings (gap opening penalty = 10 (for nucleotides) / 10 (for proteins) and gap extension penalty = 0.5 (for nucleotides) / 0.5 (for proteins)) can be employed. For nucleotides the default scoring matrix used is EDNAFULL and for proteins the default scoring matrix is EBLOSUM62.

As used herein the term "enantiomer" refers to one of two stereoisomers of a given molecule that are mirror images of each other that are non-superposable (not identical). A single chiral atom or similar structural feature in a compound causes that compound to have two possible structures which are non-superposable, each a mirror image of the other.

As used herein the term "racemate" or "racemate substrate" refers to a mixture of two stereoisomers of a chiral molecule. In most cases a racemat has equal amounts of the two stereoisomers of a chiral molecule.

The term "complementary" as used herein refers to two nucleic acid molecules that can form specific interactions with one another. In the specific interactions, an adenine base within one strand of a nucleic acid can form two hydrogen bonds with thymine within a second nucleic acid strand when the two nucleic acid strands are in opposing polarities. Also in the specific interactions, a guanine base within one strand of a nucleic acid can form three hydrogen bonds with cytosine within a second nucleic acid strand when the two nucleic acid strands are in opposing polarities. Complementary nucleic acids as referred to herein, may further comprise modified bases wherein a modified adenine may form hydrogen bonds with a thymine or modified thymine, and a modified cytosine may form hydrogen bonds with a guanine or a modified guanine.

As used herein the term "specifically hybridize" refers to a reaction of the nucleic acid sequence in question in a hybridization solution containing 0.5 M sodium phosphate buffer, pH 7.2 containing 7% SDS, 1 mM EDTA and 100 mg/ml of salmon sperm DNA at 65°C for 16 hours and washing twice at 65°C for twenty minutes in a washing solution containing 9.5xSSC and 0.1% SDS.

As used herein the term "enantiomeric excess" or "ee-value" refers to a measurement for the purity of chiral substances. It reflects the degree to which a sample contains one enantiomer in greater amounts than the other. The ee value is calculated based on the masses of both enantiomers in the sample according to the following formula: ee = (mass of enantiomer 1 - mass of enantiomer 2) / (mass of enantiomer 1 + mass of enantiomer 2) * 100. In this example enantiomer 1 is the wanted enantiomer. E.g. a sample with 70 g of enantiomer 1 and 30 g of enantiomer 2 has an ee of 40%. A racemic mixture has an ee of 0%, while a single completely pure enantiomer has an ee of 100%.

Methods for determining the ee-value are known in the art. It can e.g. be determined using gas or liquid chromatography devices, equipped with a chiral column, respectively.

When referring to the fact that an enzyme while catalyzing a reaction selectively generates the S-or the R enantiomer of a given product the terms asymmetric reaction and enantioselectivity can be used. In other words, via selective catalysis an enantiomeric excess of either the R- or the S- enantiomer is generated depending on the preference of the enzyme.

In a preferred embodiment of the isolated nucleic acid fragments encoding an oxygenase subunit (StyA) and a reductase subunit (StyB) wherein the polypeptides encoded for by the nucleotide sequence for the oxygenase subunit (StyA) and the nucleotide sequence for the reductase subunit (StyB), together have activity towards chiral arylsulfides said nucleic acid fragments are selected from the group
a) nucleic acid fragment comprising a nucleotide sequence of at least 80% sequence identity to SEQ ID NO 1 and a nucleotide sequence of at least 80% sequence identity to SEQ ID NO 2
b) nucleic acid fragment comprising a sequence complementary to SEQ ID NO 1 and a nucleic acid fragment comprising a sequence complementary to SEQ ID NO 2
c) nucleic acid fragment comprising a sequence which specifically hybridizes to said nucleic acid fragment of a) or said complementary of b).
the nucleic acid fragment comprising a nucleotide sequence with sequence identity to SEQ ID NO 1 has at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 1 and
the nucleic acid fragment comprising a nucleotide sequence with sequence identity to SEQ ID NO 2 has at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 2.

In a further preferred embodiment, the isolated nucleic acid fragments described herein consist of
a) a nucleotide sequence of at least 80% sequence identity to SEQ ID NO 1 and a nucleotide sequence of at least 80% sequence identity to SEQ ID NO 2, respectively
b) a nucleic acid fragment comprising a sequence complementary to SEQ ID NO 1 and a nucleic acid fragment comprising a sequence complementary to SEQ ID NO 2, respectively
c) a nucleic acid fragment consisting of a sequence which specifically hybridizes to said nucleic acid fragment of a) or said complementary of b).

It is preferred that in said isolated nucleic acid fragments consisting of
a) a nucleotide sequence of at least 80% sequence identity to SEQ ID NO 1 and a nucleotide sequence of at least 80% sequence identity to SEQ ID NO 2 , respectively
b) a nucleic acid fragment comprising a sequence complementary to SEQ ID NO 1 and a nucleic acid fragment comprising a sequence complementary to SEQ ID NO 2, respectively
c) a nucleic acid fragment consisting of a sequence which specifically hybridizes to said nucleic acid fragment of a) or said complementary of b)
the nucleic acid fragment consisting of a nucleotide sequence with sequence identity to SEQ ID NO 1 has at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 1 and
the nucleic acid fragment consisting of a nucleotide sequence with sequence identity to SEQ ID NO 2 has at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 2.

In a further aspect what is described herein relates to a recombinant expression vector comprising the isolated nucleic acid fragments described herein, wherein said recombinant expression vector is selected from the group
a) recombinant expression vector comprising the isolated nucleic acid fragments of claim 1 as well as a lac repressor comprising a nucleotide sequence of at least 80% sequence identity to SEQ ID NO 3
b) recombinant expression vector comprising a nucleotide sequence of at least 80% sequence identity to SEQ ID NO 4
c) recombinant expression vector comprising a sequence complementary to SEQ ID NO 4
d) recombinant expression vector comprising a sequence which specifically hybridizes to said nucleic acid fragment of b) or said complementary of c).

Surprisingly it was found that the isolated nucleic acid fragment described herein is expressed especially well from a vector comprising the lac repressor sequence.

The term "vector" or "recombinant expression vector" or "expression vector", as used herein, refers to a molecular vehicle used to transfer foreign genetic material into a cell. The vector itself is generally a DNA sequence that consists of an insert (sequence of interest) and a larger sequence that serves as the "backbone" of the vector. The purpose of a vector to transfer genetic information to another cell is typically to isolate, multiply, or express the insert in the target cell.

It is known in the art that instead of using one expression vector for gene expression two or more expression vectors can be employed or that isolated nucleic acid fragments to be expressed in a given host cell can be inserted into the genome of said cell. Moreover, combinations of these methods can be used.

In a further preferred embodiment the recombinant expression vector comprising the isolated nucleic acid fragments described herein is selected from the group
a) recombinant expression vector comprising the isolated nucleic acid fragments described above as well as a lac repressor comprising a nucleotide sequence of at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 3,
b) recombinant expression vector comprising a nucleotide sequence of at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 4
c) recombinant expression vector comprising a sequence complementary to SEQ ID NO 4
d) recombinant expression vector comprising a sequence which specifically hybridizes to said nucleic acid fragment of b) or said complementary of c).

In a preferred embodiment of the isolated nucleic acid fragments described herein, the nucleotide sequence for the oxygenase subunit (StyA) codes for a polypeptide with at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 5 and wherein the nucleotide sequence for the reductase subunit (StyB) codes for a polypeptide with at least 80% sequence identity, more preferably at least 85% sequence identity, more preferably at least 90% sequence identity, more preferably at least 95% sequence identity, more preferably at least 95%, 96%, 97%, 98%, 99%, and most preferably 100% sequence identity to SEQ ID NO 6.

A skilled person is aware that the polypeptides characterized herein via their amino acid sequence, can be encoded for by different nucleic acids sequences. This is a result of the degeneracy of the genetic code

As a result of this degeneracy of the genetic code, amino acids can be encoded by one or more codons. In different organisms, the codons coding for an amino acid are used at different frequencies. Adapting the codons of a coding nucleic acid sequence to the frequency of their use in the organism in which the sequence to be expressed is to be integrated may contribute to an increased amount of translated protein and/or to the stability of the mRNA in question in the particular plant cells or plants. The frequency of use of codons in the host cells or hosts in question can be determined by the person skilled in the art by examining as many coding nucleic acid sequences of the organism in question as possible in terms of the frequency with which certain codons are used for coding a certain amino acid. The frequency of the use of codons of certain organisms is known to the person skilled in the art and can be determined in a simple and rapid manner using specifically developed algorithms implemented into computer programs (e.g. Grote et al., 2005, Nucleic Acids Research 33, W526-W531; doi: 10.1093/nar/gki376). Tools using such algorithms are publicly accessible and are provided for free as web-interfaces inter alia on the World Wide Web from various institutions, like the European Bioinformatics Institute (EMBL-EBI) and others (for example http://www.jcat.de; http://gcua.schoedl.de/; http://www.kazusa.or.jp/codon/; http://www.entelechon.com/eng/cutanalysis.html; http://www.ebi.ac.uk/Tools/st/emboss_backtranseq/). Adapting the codons of a coding nucleic acid sequence to the frequency of their use in an organism in which the sequence is intended to be expressed can be carried out by in vitro mutagenesis or, preferably, by de novo synthesis of the gene sequence. Methods for the de novo synthesis of nucleic acid sequences are known to the person skilled in the art. A de novo synthesis can be carried out, for example, by initially synthesizing individual nucleic acid oligonucleotides, hybridizing these with oligonucleotides complementary thereto, so that they form a DNA double strand, and then ligating the individual double-stranded oligonucleotides such that the desired nucleic acid sequence is obtained. The de novo synthesis of nucleic acid sequences including the adaptation of the frequency with which the codons are used to a certain target organism can also be sourced out to companies offering this service (for example Eurofins MWG).

In a further aspect what is described herein relates to the use of the oxygenase subunit (StyA) described herein together with the reductase subunit (StyB) described herein for the enantioselective oxidation of a compound according to formula I 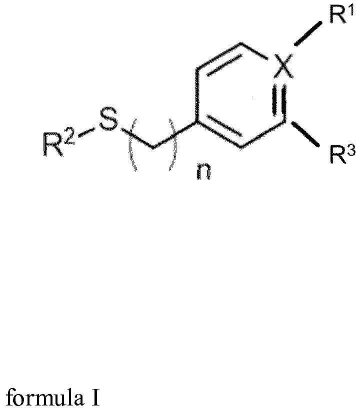
wherein X is C or N, and
wherein if X is N, R₁ is absent
and if X is C, R₁ is selected form the group consisting of a H, NO₂, a halogen, NH₂ , an C₁ to C₆ alkyl, or an an C₁ to C₆ O-alkyl, and
wherein n is 0 or 1, and
wherein R² is an C₁ to C₆ alkyl and
wherein R³ is a halogen or H
into the S -sulfynil-enantiomer.

As used herein the term "alkyl" refers to a linear or branched, substituted or unsubstituted, saturated or unsaturated or but not cyclic hydrocarbon chain.

In a preferred embodiment, the alkyl is a linear, unsubstituted, saturated hydrocarbon chain.

In an especially preferred embodiment the alkyl is an ethyl or methyl group.

In a further preferred embodiment R₁ is selected form the group consisting of H, NO₂, a halogen, an C₁ to C₆ alkyl, or an an C₁ to C₆ O-alkyl.

Preferably, the halogen is selected from fluor, bromide or chloride,

In one embodiment the compound of formula I is selected from the group consisting of phenyl methyl sulfide, 4-methylphenyl methyl sulfide, 4-fluorophenyl methyl sulfide, 4-chlorophenyl methyl sulfide, 4-bromophenyl methyl sulfide, 4-methoxyphenyl methyl sulfide, 4-nitrophenyl methyl sulfide, ethyl phenyl sulfide, benzyl methyl sulfide, phenyl vinyl sulfide, 2-chloro-4-(methylsulfanylmethyl)pyridine.

In an especially preferred embodiment the compound of formula I is 2-chloro-4-(methylsulfanylmethyl)pyridine.

In a further aspect what is described herein relates to a method for the enantioselective oxidation of a compound according to formula I into the S-sulfynil-enantiomer comprising
- providing said compound according to formula I,
- providing the oxygenase subunit (StyA) described herein together with the reductase subunit (StyB) described herein as polypeptides,
- reacting said compound according to formula I with said polypeptides for 2-48 hours at a pH of 5-9 and a temperature of 15-40°C.

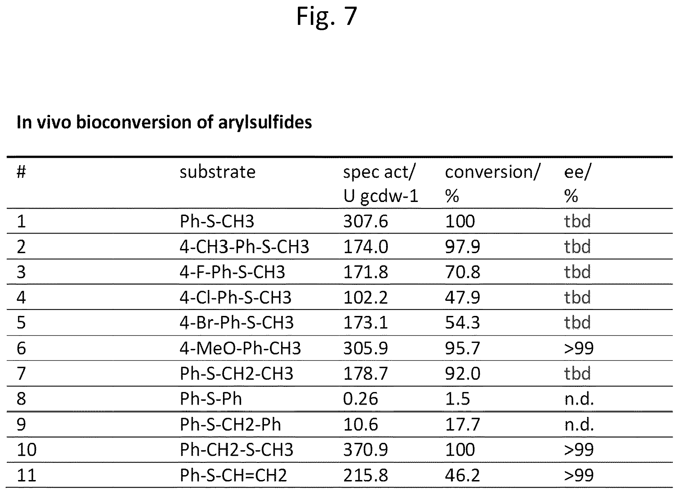

This method allows the enantioselective generation of the S-enantiomer of the oxidized compound according to formula I with a high yield in a short time period (cf. FIG.7). In other words, it was surprisingly found that the oxygenase subunit (StyA) described herein together with the reductase subunit (StyB) described herein through oxidizing the compound according to formula I selectively generate the S-enantiomer.

As stated above the reaction is carried out for 0.5-48 hours at a pH of 5-9 and a temperature of 15-40°C, preferably for 1-24 hours at a pH of 6-8 and a temperature of 25-35°C, most preferably for 1-4 hours at a pH of 7.2 and a temperature of 30°C.

The method described herein can be carried out via providing the polypeptides described herein as mixture of the purified oxygenase subunit (StyA) and the purified reductase subunit (StyB), as cell-free enzyme extract or through providing whole cells expressing both the oxygenase subunit (StyA) and reductase subunit (StyB) .

In a preferred embodiment of the method described herein, the method further comprises the step of providing the S-enantiomer of the oxidized compound according to formula I, i.e. purifying said S-enantiomer of the oxidized compound according to formula I.

In an embodiment of the method described herein, the oxygenase subunit (StyA) described herein and the reductase subunit (StyB) described herein are expressed in recombinant cells provided in a cell culture medium and the compound according to formula I is directly added to the cell culture.

This method employing whole-cell biocatalysis has the advantage that the product i.e. S-enantiomer of the oxidized compound according to formula I was generated in very high quantities (cf. FIG. 3 and FIG. 4) significantly exceeding production rates of whole cell biocatalysts reactions described so far (cf. FIG. 5). Moreover, as shown below the achieved specific activity and the resulting STY in the whole cell biocatalysis system was several orders of magnitude higher than the use of cell lysate.

| Conditions | Cofactor recycling | Further additives | Specific activity |
|---|---|---|---|
| Cell free extract | GDH based system | NAD, FAD, Glucose | 0.7 U / g CDW |
| Whole cell system | Whole cells | | 450 U / g CDW |

For cofactor dependent enzymes such as the oxygenase subunit (StyA) described herein another advantage of catalysis reaction in whole cells is the ability of the microbial metabolism for in vivo cofactor regeneration. This is especially an advantage as stoichiometric addition of the cofactor NAD(P)H is not economically.

Although whole-cell bioprocesses have been described for oxyfunctionalizations (Schrewe et al., 2013), the inventors of the current invention have shown for the first time that chiral arysulfoxides - i.e. in this case only the S-enantiomer of the oxidized compound according to formula I - can be generated efficiently enough, enantiomerically pure enough and in suitable quantities for synthesis on a production scale using an SMO - i.e. the polypeptides described herein - as whole cell biocatalysts.

As used herein the term "biocatalysis" refers to a reaction catalyzed by enzymes.

As used herein the term "whole-cell biocatalysis" refers to a catalysis in living cells by enzymes that said are recombinantly expressed by said cells.

In a preferred embodiment of the method employing whole-cell biocatalysis described herein, the oxygenase subunit (StyA) described herein and the reductase subunit (StyB) described herein are expressed in recombinant cells provided in a cell culture medium, the compound according to formula I is directly added to the cell culture medium once the cells have reached a predetermined cell density and wherein the product, i.e. the S-enantiomer of the oxidized compound according to formula I accumulates in the cell culture medium.

The term "cell viability" as used herein refers to the ability of cells in culture to survive under a given set of culture conditions or experimental variations. The term as used herein also refers to that portion of cells which are alive at a particular time in relation to the total number of cells, living and dead, in the culture at that time.

The term "cell density" as used herein refers to that number of cells present in a given volume of medium.

A person skilled in the art knows how to determine the cell viability and the cell density. One example of measuring cell density is via measuring absorbance, or optical density, of a sample at a wavelength of 600 nm. It is a common method for estimating the concentration of bacterial or other cells in a liquid. Measuring the concentration can indicate the stage of cultured cell population, i.e. whether it is in lag phase, exponential phase, or stationary phase.

In one embodiment of said aspect the employed recombinant cells are selected from the group consisting of *E.coli, Pseudomonas, Corynebacterium, Bacillus, Saccharomyces, Pichia* and the wildtype strain *A baylyi.*

In a preferred embodiment of said aspect the employed recombinant cells are *E.coli* selected from the group comprising of JM 101, MG1655 and W3110, DH5α, DH10B; JM109, BL21(DE3).

In one embodiment of said aspect the recombinant cells expressing the oxygenase subunit (StyA) described herein and the reductase subunit (StyB) described herein, i.e. the styrene monooxygenase from *A. baylyi,* are cultured until a predetermined cell density is reached, then the cells are harvested, washed and returned to the culture vessel. Thus, the subsequent biocatalysis is carried out in living cells which have stopped growing. Additionally or alternatively none or too little MgSO₄ is added to the cell culture medium. Hence, the cells stop growing once the MgSO₄ has been consumed. Other limitations can be introduced via limiting the supply of nitrogen, phosphate and/or sulphur.

Thus, in a preferred embodiment of said aspect the recombinant cells expressing the oxygenase subunit (StyA) described herein and the reductase subunit (StyB) described herein, are grown until a cell density of 0.5-2 is reached at OD 600, preferably until a cell density of 0.6 is reached at OD 600 and then the cells are harvested and washed and/or Mg supply is limited.

In a preferred embodiment of the method described herein the compound according to formula I is added continuously or at regular time intervals at a concentration, which is below the conversion rate of the oxygenase subunit (StyA) and the reductase subunit (StyB) described herein.

In other words, the concentration of the compound according to formula I is lower than the maximum rate at which the oxygenase subunit (StyA) and the reductase subnit (StyB) can process the compound according to formula I. Hence, before the compound according to formula I can accumulate it is already converted to the product. This is especially advantageous, if the compound according to formula I is toxic to the cells at higher concentrations.

A person skilled in the art knows how to determine the maximum rate at which the enzyme can process the substrate and thus how much substrate can be added to a given reaction continuously or in a given time interval. To understand the toxicity of educt and product the growth rate of the production strain is determined in presence of different educt and product concentration.

For example in the case of the enantioselectve oxidation of 2-chloro-4-(methylsulfanylmethyl)pyridine to 2-Chloro-4-((methyl -S-sulfinyl)methyl)pyridine in E. coli cells using the oxygenase subunit (StyA) described herein and the reductase subunit (StyB) described herein, it was found that a substrate concentration above 10 mM entirely inhibited the growth of the cells. The toxicity of the product was significantly lower.

Thus the amount of educt, which is added per time interval is determined by the toxicity of the educt and the maximum conversion rate of the cells. By way of example cells with a total activity of 450 U / gcwd (cell weight dry) are used. For cells of this activity the maximum rate at which potentially toxic educt can be added without harming the cells would in theory thus be around 450 µmol/min. To be on the safe side a feeding rate which corresponds to a conversion rate of 150 U / gcdw is hence usually employed. However, the feeding rate can be raised to a level corresponding to an activity of 300 U/ gcdw without harming the cells. As the activity of the cells in this example can also be higher up to 600 U / gcdw and lower (150 U/ gcdw) .The feed rate has to be adapted accordingly .

The very high activity of the oxygenase subunit (StyA) described herein and the reductase subunit (StyB) described herein at low substrate concentrations is the prerequisite for the possibility to add the compound according to formula I continuously or in regular time intervals.

The whole cell biocatalysis can be carried out in aqueous medium - also termed "one phase system" - or in a combination of aqueous and organic media - also termed "two phase system". Organic media have been developed for biocatalysis as a consequence of the poor solubility in aqueous media of many organic compounds of commercial interest which can potentially be transformed by enzymes or microorganisms. The advantages of aqueous/apolar medium two-phase systems, however, include not only the production of sparingly water-soluble compounds, but also the maintenance of a low concentration of toxic or inhibitory compounds in the aqueous phase. Typical organic phases are: DEHP: Di(2-ethylhexyl)phthalat or Bis(2-ethylhexyl)phthalate, DINP (Diisononylphthalat), DBP (Dibutylphthalat) and DIBP (Diisobutylphthalat). The following second phases can be used as well: medium and longchain alkans (C10-C16), long chain alkylic alcohols (C12-C14), esters of long chain fatty acids such as ethyloleat, butyloleat, butylstearate, alcohols of unsaturated fatty acids (oleyalkohol), long chain fatty acids (C10-C12), oleic acid, silicon oil, olive oil and corn oil.

In general there are several options to further optimize the whole cell biocatalysis described above.

For example the biocatalyst reaction can be carried out in cells showing an even higher expression rate of the polypeptides described herein. This higher expression rate leads to a higher enzymatic activity and therefore higher yields. In order to protect the biocatalyst reaction from the influence of highly reactive oxygen species produced in those high yielding cells additional detoxifying enzymes such as catalase, superoxyd dismutase or glutathion peroxydase can beco expressed with the polypeptides described herein.

Moreover, depending on the interaction of the polypeptides described herein and the metabolism of the host cell it can also be expedient to adapt the expression level in order to achieve a well balance conversion rate of all contributing enzymes.

Therefore alternatively or in addition, a higher STY is possible in case of a soluble or volatile product, via continuously removing the potentially toxic product. This can be achieved for example through the use of a continuous culture plus cell retention or a permanent binding of the product to a resin. In case of a volatile product said volatile product can be removed via the gas phase.

In a further aspect what is described herein refers to the use of the method described herein to provide the S-enantiomer of the oxidized compound according to formula I with an ee-value of more than 95%.

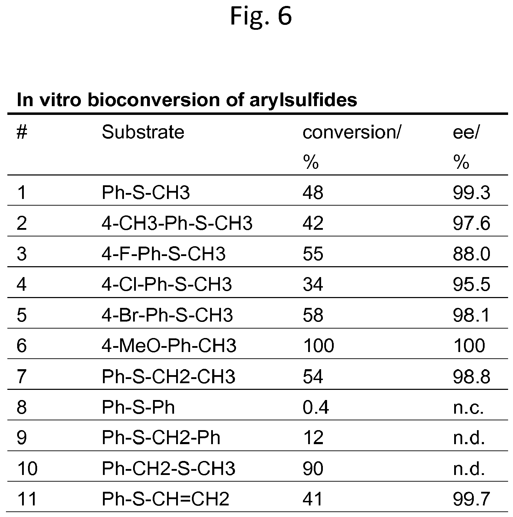

It was surprisingly found that in addition to its remarkable high activity the oxygenase subunit (StyA) described herein and the reductase subunit (StyB) described herein generate the S-enantiomer of the oxidized compound according to formula I with remarkably enantiomeric excess rates e.g. of more than 95% and partly even more than 99% both in vitro and in vivo (cf. FIG. 6 and FIG. 7).

This is an important finding, as typically for pharmaceutical agents, a minimum enantiomeric purity of 98 % enantiomeric excess (ee) is a prerequisite and enantiomeric ratios above 98% are difficult to obtain both enzymatically and via chemical synthesis.

Moreover, this result was also unexpected since as mentioned above all styrene monooxygenases described so far have a very limited substrate tolerance.

In another preferred embodiment of said use the S-enantiomer of the oxidized compound according to formula I is generated at a rate of 10 to 60 g/l x h, even more preferably at a rate of 20 to 50 g/l x h and most preferably at a rate of 30 - 40 g/l x h.

In an especially preferred embodiment of said use 2-chloro-4-((methyl -S-sulfinyl)methyl)pyridine is generated enantioselective from 2-chloro-4-(methylsulfanylmethyl)pyridine at a rate of 30 - 40 g/l x h.
Thus, the selective oxidation to only the S-enantiomer of the compounds according to formula I in combination with its high activity renders the polypeptides described herein very interesting for a wide variety of applications in synthesis of chiral sulfoxides.

In addition, the use of enzymes in chemical synthesis usually reduces waste.

Taken together these findings render the polypeptides described herein suitable for synthesis on a production scale.

### FIGURES

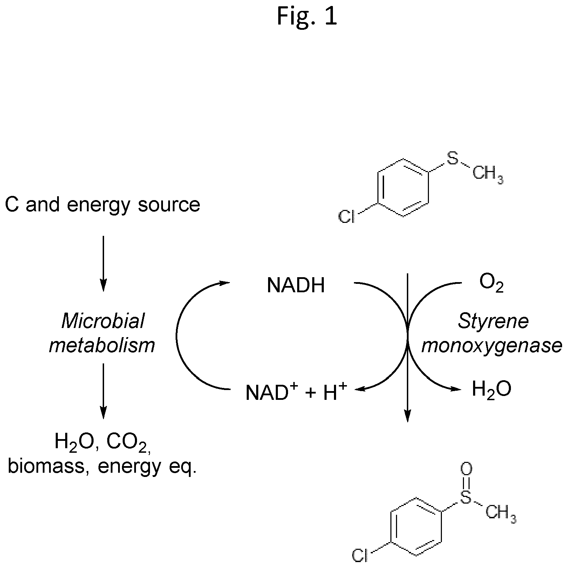

FIG. 1: Figure 1 shows a schematic overview of the reaction of one embodiment of the invention. In this case the substrate is 4-chlorophenyl methyl sulfide. The substrate is added to a cell culture, the reaction takes place inside the cells and the reaction product accumulates in the cell culture medium.

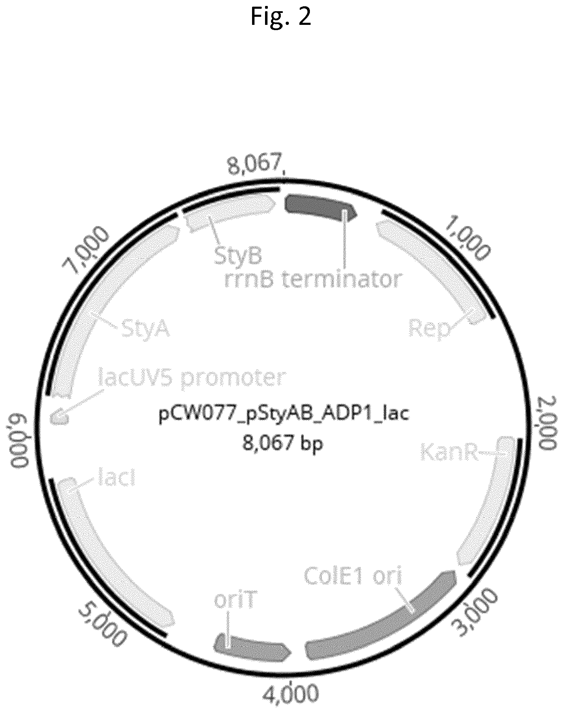

Fig. 2 Figure 2 depicts a schematic illustration of the employed pStyAB_ADP1_lac vector.

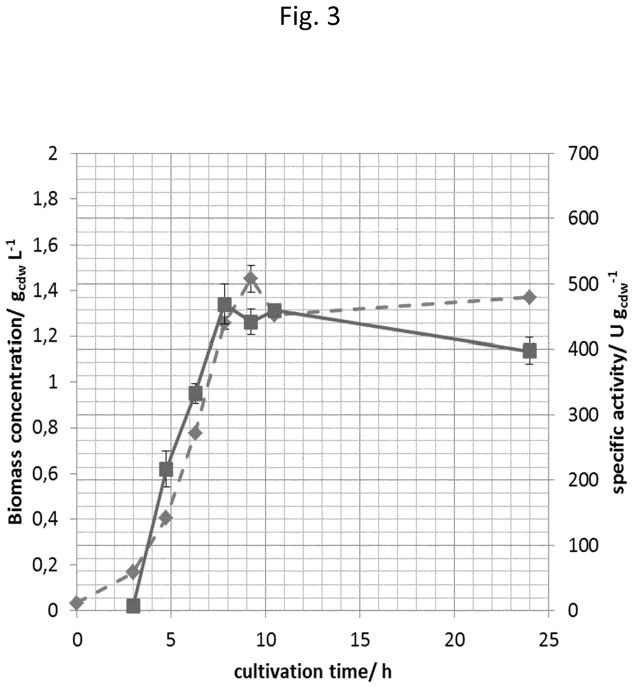

FIG 3 Figure 3 shows the specific activity of the styrene monoxygenase from A. baylyi expressed from the pStyAB_ADP1_lac vector (squares) and microbial growth (diamonds) in dependency of the cultivation time.

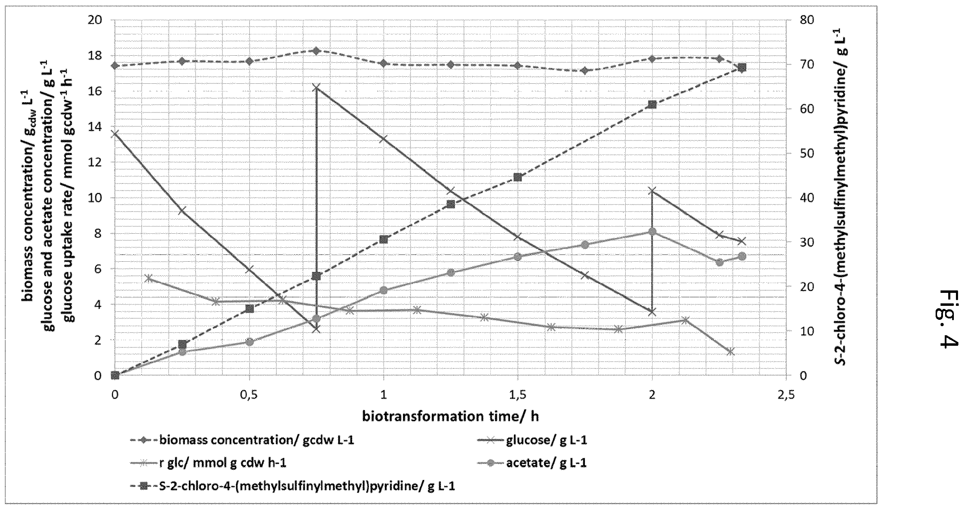

FIG. 4 The graph of Figure 4 demonstrates that product accumulated to very high concentrations exceeding 62 g L-1 after 2.3 h of biotransformation. No substrate accumulation was observed within the investigated time period

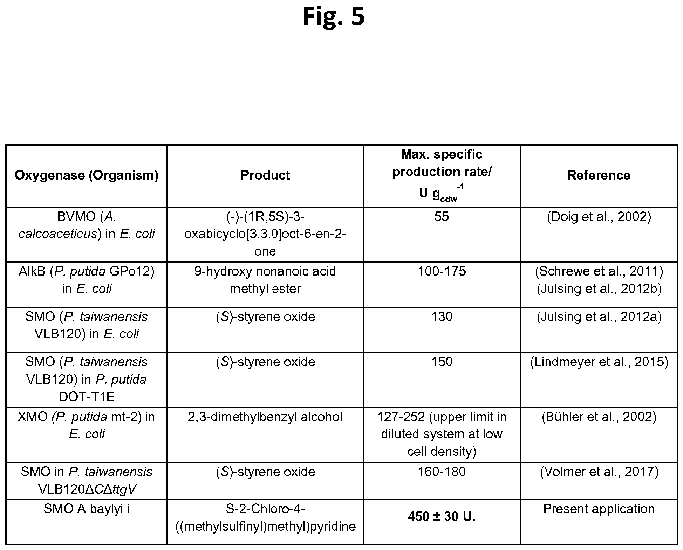

FIG 5: The table of Figure 5 demonstrates that the maximal specific production rate of the whole cell biocatalysis was surprisingly considerably higher than it could be expected from the literature.
FIG. 6 The Table of Figure 6 demonstrates that the styrene monooxygenase A. baylyi described herein converts a variety of different substrates in vitro.
FIG. 7 The Table of Figure 7 demonstrates that the styrene monooxygenase of A. baylyi described herein converts a variety of different substrates in vivo.

### WENN ÜBERTRAGUNSVERTRAG AUS LEIPZIG VORLIEGT PRUFEN OB DIESE ERGEBNISSE ENTHALTEN SIND

FIG. 8 Figure 8 shows the key parameters for the characterization and quantification of cell-free and whole-cell processes as published by Schrewe et al. 2013.

### EXAMPLES

The following examples are illustrative and not limiting. One of skill will recognize a variety of non-critical parameters that can be altered to achieve essentially similar results.

### 1) Identification and characterization of a novel styrene monooxgenase

Gene sequences of known SMOs and monooxygenases were screened for promoter and or ribosome entry sites in order to detect potentially functional enzymes. In the next step the different SMOs were expressed e.g. in E. coli. Moreover, structural models of the different SMOs were created and the different SMOs were screened for activity with the potential substrate 2-chloro-4-(methylsulfanylmethyl)pyridine. Surprisingly, it was found that the isolated nucleic acid fragments encoding an oxygenase subunit (StyA) and a reductase subunit (StyB) respectively described herein was able to generate the (S)-enantiomer of - 2-chloro-4-(methylsulfinylmethyl)pyridine in high enantiomeric excess.

In detail, the isolated nucleic acid fragments encoding an oxygenase subunit (StyA) and a reductase subunit (StyB), respectively described herein from *A. baylyi* were expressed and it was demonstrated that chiral sulfoxides are generated using the substrates given in the table below In order to do so, the StyA unit from Acinetobacter sp. ADP1 was cloned into a pET-vector inducible with IPTG and expressed in *E. coli* BL21 pLysS grown in LB medium (10 g/l tryptone, 5 g/l yeast extract, 10 g/l or 20 g/l NaCl, 100 µg/ml ampicillin, 50 µg/ml Chloramphenicol). For the expression a 5 ml overnight culture was used as inoculum for a fermenter with 31 medium or a flask with 500 ml medium. The cells were cultured at 37°C until the culture reached an optical density of 0.6 which was the threshold for induction of recombinant gene expression using IPTG (0,1 mM). The culture was performed for another 18h at 20°C. During this time the medium turned blue, if the expression was successful due to indigo formation. The cells were harvested and the cellular walls were disrupted using a French press. The protein was located in the soluble part of the cell extract, which was separated via centrifugation (45 min at >20000xg). The target protein was purified for the subsequent characterization.

The StyB unit from *Acinetobacter* baylyi sp. ADP1 was expressed in a similar fashion.

In order to test the activity the StyB unit (reductase) was employed in excess to supply the StyA unit (oxygenase) with sufficient reduced FAD. Therefore, NADH was produced by a format-dehydrogenase from format and NAD in the respective enzymatic reaction.

The enzyme conversion rate was probed every minute for 15 min for the kinetic analysis. If possible, i.e. soluble, 2 mM substrate were employed. In case of clouding 1 mM substrate was employed. The substrate was dissolved in ethanol. Reactions in the probes were stopped with a 1:1 mixture of ice-cold methanol and acetonitrile and afterwards analyzed using HPLC.

The educt and the product were analyzed on an Agilent 1100 HPLC device.

The results are shown in the table below:

| Substrat | yield%) | *ee %* | Abs.confi. |
|---|---|---|---|
| Ph-S-CH3 | 48 | 99.3 | *S* |
| *p*(Cl)-Ph-S-CH3 | 34 | 95.5 | *S* |
| *p*(Br)-Ph-S-CH3 | 58 | 98.1 | *S* |
| *p*(F)-Ph-S-CH3 | 55 | 99.0 | *S* |
| *p*(CH3)-Ph-S-CH3 | 42 | 97.6 | *S* |
| Ph-S-CH=CH2 | 40 | 99.7 | *S* |

### 2) Whole cell biocatalysis using the novel SMO

In order to further increase production rates it was tried to generate the product, i.e. the S-enantiomer of -2-chloro-4-(methylsulfinylmethyl)pyridine using a whole cell bio-catalysis in a minimal medium employing live cells even though the substrate is potentially toxic. Ideally, slowly growing or no-growing cells, i.e. resting but metabolically active cells, were used. To further enhance the enantiomeric excess (ee) value a novel vector, the pCom10:lac Vector, was developed.

In detail, the styrene monooxygenase subunits StyA and StyB were expressed in E. coli JM101 cultivated in M9 minimal medium from the pStyAB_ADP1_lac vector. To find the maximum specific activity samples were taken after previously defined time points after addition of the inducer (1 mM IPTG). Cells were retrieved from the actively growing culture and used for the determination of the resting cell activity. As shown in FIG. 3 the specific activity increased steadily from the time point of induction and reached a maximum of 450 ± 30 U/gcdw 4.8 h after induction.

The success of the biotransformation was assessed based on substrate conversion, product formation, and enantiomeric excess (ee) determined by chiral HPLC and GC.

In detail, the specific activity was quantified as follows: StyA and StyB were expressed in E. coli JM101 carrying the pStyAB_ADP1_lac vector. Cells were cultivated in M9 minimal medium and induced at an OD of 0,6 - 0,8. After 3 - 5 hours the cell were harvested, washed in PP buffer and resuspended in the same buffer containing glucose Then 750 µl of cells (diluted if needed) were taken and incubated in 2 ml tubes on a thermomixer set to 30 °C and 1500 rpm. Cell suspensions were pre-warmed for 5 min and then 2 mM of the substrate was added from 100-fold concentrated isopropanol stock solutions. After desired time points, 40 µL of 20% (w/v) perchloric acid was added to quench the reaction. The whole-cell biocatalyst is inactivated directly upon addition of perchloric acid that leads to a pH shift from pH 7.4 to 2.0 The addition of perchloric acid had no effect on the product itself, however, the protein immediately precipitates and was separated via centrifugation.

Successful separation of the product i.e. the S-enantiomer of -2-chloro-4-(methylsulfinylmethyl)pyridine was achieved on a Dionex UltiMate 3000 HPLC system (Thermo Scientific) equipped with a non-polar HPLC column with C18 matrix (Accucore C18, 3 x 150 mm, 2.6 µm particle size, Thermo Scientific). The oven was set to 30°C and the analytes were eluted isocratically (0-6 min) 80% 10 mM ammonium phosphate buffer and 20% ACN and a subsequent (6-18 min) gradient to 90% ACN. Afterwards the column was re-equilibrated 2 min at 80% 10 mM ammonium phosphate buffer and 20% ACN. 2 µL of the respective sample were injected. The organic compounds were detected by absorption using diode array detector (DAD) at a wavelength of 210 nm. The sample preparation for analytical standards was performed as follows: 5 µL of 100-fold concentrated isopropanol stock solutions of educt or product were added to 495 µL potassium phosphate buffer (pH 7.4, 0.1 M) and subsequently 500 µL of a 1 to 1 (v/v) mixture of ACN and MeOH were added. The sample was centrifuged after mixing and directly subjected to HPLC analysis.

In the next step a preparation on technical scale was conducted.

A technical scale bioreactor (3 L total volume) experiment was performed to produce the product on a larger scale. For this, *E. coli* JM101 (pStyAB_ADP1_lac) was cultivated in a 3 L stirred tank reactor equipped with two Rushton impellers. The working volume was set to 2 L. The cells were cultivated in M9 minimal medium in batch mode overnight (12 h, 1.5 % (w/v) glucose) followed by a glucose limited fed-batch phase (6 h). The pH was controlled at 7.2 by titration with 15% phosphoric acid and 25% (v/v) ammonium hydroxide. The gene expression of the subunits StyA and StyB was induced 1 h after start of the fed batch phase with IPTG. The growth rate was set to ∼0.18 h¹ in order prevent acetate formation and to reach a cell density of approximately 20 gcdw L⁻¹ after 6 h of glucose feed. Subsequently, the cells were harvested by centrifugation and resuspended in 2 L 0.1 M potassium phosphate buffer (pH 7.4). The specific activity of the whole cell biocatalyst was determined in separate resting cell assays. Only 1.5% glucose was added to the bioreactor to provide the living cells with energy. A constant educt feed (∼0.82 g min-1) i.e. a feed with educt (2-chloro-4-(methylsulfanylmethyl)pyridine was started 5 min later. This educt feed ensured that the available specific activity of the whole cell biocatalyst was so high that the added educt was immediately converted into the less toxic product product i.e. the S-enantiomer of -2-chloro-4-(methylsulfinylmethyl)pyridine. Samples for biomass, product/substrate and glucose/acetate were retrieved every 15 minutes.

This approach led to an efficient product formation without detectable substrate accumulation until the solubility of the product was exceeded.

As shown in FIG. 4 product accumulated to surprisingly high concentrations exceeding 62 g L⁻¹ after 2.3 h, i.e. 30 g L⁻¹ per hour of biotransformation. No substrate accumulation was observed within the investigated time period. Product was analyzed as described above.

### 3) Variety of substrates that can be employed

In addition it was surprisingly found that the polypeptides described herein does not only accept 2-chloro-4-(methylsulfanylmethyl)pyridine as substrate, but converts a variety of different substrates with excellent enantioselectivity. The observed efficiency of the biocatalyst with respect to activity and extremely high ee values was unexpected, since the biocatalysts that have been shown to form enantiopure sulfoxides from the corresponding sulfides so far were strongly dependent on the arylsulfide applied (Rioz-Martínez et al., 2010, Adam et al., 2005). In detail, the results presented in FIG. 6 and FIG. 7 demonstrate that the claimed enzyme converts a variety of different substrates with excellent enantioselectivity both in vitro and in vivo using biotransformation.

### REFERENCES

- Adam W, Heckel F, Saha-mo CR, Taupp M, Meyer J, Schreier P. 2005. Opposite Enantioselectivities of Two Phenotypically and Genotypically Similar Strains of Pseudomonas frederiksbergensis in Bacterial 71:2199-2202.
- Bühler B, Witholt B, Hauer B, Schmid A. 2002. Characterization and application of xylene monooxygenase for multistep biocatalysis. Appl. Environ. Microbiol. 68:560-568.
- Doig SD, Avenell PJ, Bird PA, Gallati P, Lander KS, Lye GJ, Wohlgemuth R, Woodley JM. 2002. Reactor operation and scale-up of whole cell Baeyer-Villiger catalyzed lactone synthesis. Biotechnol. Prog. 18:1039-1046.
- Julsing MK, Kuhn D, Schmid A, Bühler B. 2012a. Resting cells of recombinant E. coli show high epoxidation yields on energy source and high sensitivity to product inhibition. Biotechnol. Bioeng. 109:1109-1119.
- Julsing MK, Schrewe M, Cornelissen S, Hermann I, Schmid A, Bühler B. 2012b. Outer membrane protein AlkL boosts biocatalytic oxyfunctionalization of hydrophobic substrates in Escherichia coli. Appl. Environ. Microbiol. 78:5724-5733.
- Lindmeyer M, Meyer D, Kuhn D, Bühler B, Schmid A. 2015. Making variability less variable: matching expression system and host for oxygenase-based biotransformations. J. Ind. Microbiol. Biotechnol. 42:851-866.
- Rioz-Martínez A, De Gonzalo G, Pazmiño DET, Fraaije MW, Gotor V. 2010. Enzymatic synthesis of novel chiral sulfoxides employing Baeyer-villiger monooxygenases. European J. Org.
- Sadauskas, M., Vaiteku̅nas, J. , Gasparav̌iciu̅tė, R., Meškys, R., (2017) Genetic and Biochemical Characterization of Indole Biodegradation in Acinetobacter sp. Strain 0153 Appl. Environ. Microbiol. doi:10.1128/AEM.01453-17
- Schrewe M, Julsing MK, Bühler B, Schmid A. 2013. Whole-cell biocatalysis for selective and productive C-O functional group introduction and modification. Chem. Soc. Rev. 42:6346-77
- Schrewe M, Magnusson AO, Willrodt C, Bühler B, Schmid A. 2011. Kinetic Analysis of Terminal and Unactivated C□H Bond Oxyfunctionalization in Fatty Acid Methyl Esters by Monooxygenase-Based Whole-Cell Biocatalysis. Adv. Synth. Catal. 353:3485-3495.
- Tischler, D., Eulberg, D., Lakner, S., Kaschabek S., van Berkel W., Schlomann, M. (2009) Identification of a Novel Self-Sufficient Styrene Monooxygenase from Roodococcus opacus 1CP, JOURNAL OF BACTERIOLOGY, Aug. 2009, p. 4996-5009
- Tischler D, Gröning JAD, Kaschabek SR, Schlomann M. (2012) One-component 690 styrene monooxygenase: an evolutionary view on a rare class of flavoproteins. Appl. 691 Biochem. Biotechnol. 167:931-944
- Volmer J, Schmid A, Bühler B. 2017. The application of constitutively solvent-tolerant P. taiwanensis VLB120Δ C Δ ttgV for stereospecific epoxidation of toxic styrene alleviates carrier solvent use. Biotechnol. J.: 1600558..

## Claims

1. An isolated nucleic acid fragment encoding an oxygenase subunit (StyA) and an isolated nucleic acid fragment encoding a reductase subunit (StyB), wherein the polypeptides encoded for by the nucleotide sequences for the oxygenase subunit (StyA) and the nucleotide sequence for the reductase subunit (StyB) together have activity towards chiral arylsulfides and wherein said nucleic acid fragments are selected from the group
a) nucleic acid fragment comprising a nucleotide sequence of at least 80% sequence identity to SEQ ID NO 1 and a nucleotide sequence of at least 80% sequence identity to SEQ ID NO 2,
b) nucleic acid fragment comprising a sequence complementary to SEQ ID NO 1 and a nucleic acid fragment comprising a sequence complementary to SEQ ID NO 2,
c) nucleic acid fragment comprising a sequence which specifically hybridizes to said nucleic acid fragment of a) or said complementary of b).

2. A recombinant expression vector comprising the isolated nucleic acid fragments of claim 1, wherein said recombinant expression vector is selected from the group
a) recombinant expression vector comprising the isolated nucleic acid fragments of claim 1 as well as a lac repressor comprising a nucleotide sequence of at least 80% sequence identity to SEQ ID NO 3,
b) recombinant expression vector comprising a nucleotide sequence of at least 80% sequence identity to SEQ ID NO 4
c) recombinant expression vector comprising a sequence complementary to SEQ ID NO 4
d) recombinant expression vector comprising a sequence which specifically hybridizes to said nucleic acid fragment of b) or said complementary of c).

3. The isolated nucleic acid fragments of claim 1, wherein the nucleotide sequence for the oxygenase subunit (StyA) codes for a polypeptide with at least 80% sequence identity to SEQ ID NO 5 and wherein the nucleotide sequence for the reductase subunit (StyB) codes for a polypeptide with at least 80% sequence identity to SEQ ID NO 6.

4. Use of the oxygenase subunit (StyA) of claim 3 together with the reductase subunit (StyB) of claim 3 for the enantioselective oxidation of a compound according to formula I 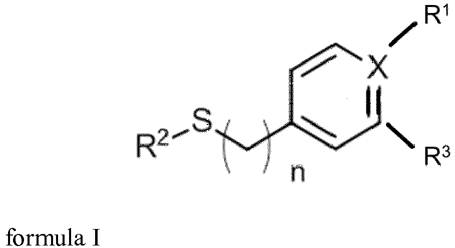
wherein X is C or N, and
wherein if X is N, R₁ is absent
and if X is C, R₁ is selected form the group consisting of a H, NO₂, a halogen, NH₂ , an C₁ to C₆ alkyl, or an an C₁ to C₆ O-alkyl, and
wherein n is 0 or 1, and
wherein R² is an C₁ to C₆ alkyl and
wherein R³ is a halogen or H
into the S -sulfynil-enantiomer.

5. Use according to claim 4 for the enantioselective oxidation of 2-chloro-4-(methylsulfanylmethyl)pyridine to 2-Chloro-4-((methyl - S - sulfinyl)methyl)pyridine

6. Method for the enantioselective oxidation of a compound according to formula I into the S-sulfynil-enantiomer comprising
• providing a compound according to formula I,
• providing the oxygenase subunit (StyA) of claim 3 together with the reductase subunit (StyB) of claim 3 as polypeptides
• reacting said compound according to formula I with said polypeptides for 2-48 hours , with a the pH is in the range of 5-9 and the temperature is in the range of 15-40 °C .

7. Method according to claim 6, wherein the oxygenase subunit (StyA) of claim 3 and the reductase subunit (StyB) of claim 3 are expressed in recombinant cells provided in a cell culture medium, the compound according to formula I is directly added to the cell culture medium once the cells have reached a predetermined cell density and wherein the product accumulates in the cell culture medium.

8. Method according to claim 7 wherein the compound according to formula I is added continuously or at regular time intervals at concentration which is below the conversion rate of the polypeptides of claim 4.

9. Use of the method according to claim 7 or 8 to provide the S-enantiomer of the oxidized compound according to formula I with an ee-value of more than 95%.

10. Use of the method according to claims 7-9 to provide the S-enantiomer of the oxidized compound according to formula I at a rate of 10 to 60 g/l x h.
